# EUROPEAN PATENT SPECIFICATION

(11) **EP 2 004 168 B1**
(45) Date of publication and mention of the grant of the patent: **03.08.2011**
(21) Application number: 07723634.7
(22) Date of filing: 27.03.2007
(51) Int. Cl.: A61K 31/185, A61K 31/215, A61K 45/06, A61K 9/28, A61P 5/16

(54) **STABLE ORAL PHARMACEUTICAL COMPOSITION CONTAINING THYROID HORMONE RECEPTOR AGONISTS**
STABILE ORALE PHARMAZEUTISCHE ZUSAMMENSETZUNG MIT THYROIDHORMONREZEPTORAGONISTEN
COMPOSITION PHARMACEUTIQUE ORALE STABLE CONTENANT DES AGONISTES DES RÉCEPTEURS DES HORMONES THYROÏDIENNES

(30) Priority: 28.03.2006 GB 0606212
(43) Date of publication of application: 24.12.2008
(73) Proprietor: KARO BIO AB, 141 57 Huddinge (SE); Bristol-Myers Squibb Company, Princeton NJ 08543-4000 (US)
(72) Inventor: GARG, Neeraj, S-141 57 Huddinge (SE); RAO, VENKATRAMANA M., Edison, NJ 08820 (US); GANDHI, Rajesh, B., Plainsboro, NJ 08536 (US); WASHBURN, William N., Titusville, NJ 08560 (US); KOEHLER, Konrad, 30161 Hannover (DE); MALM, Johan, S-142 66 Trängsund (SE)
(74) Representative: Brady, Paul Andrew
(86) International application number: PCT/EP2007/002688
(87) International publication number: WO 2007/110226

(56) References cited:
- EP-A2- 1 291 021
- WO-A-01/60784
- WO-A-02/32425
- WO-A-2004/007430
- WO-A-2004/067482
- US-A1- 2004 028 737
- YOKOYAMA N: "Synthesis and structure-activity relationships of oxamic acid and acetic acid derivatives related to L-thyronine" JOURNAL OF MEDICINAL CHEMISTRY, AMERICAN CHEMICAL SOCIETY. WASHINGTON, US, vol. 38, 1995, pages 695-707, XP002080908 ISSN: 0022-2623
- LIU YE ET AL: "Thyroid Receptor Ligands" JOURNAL OF MEDICINAL CHEMISTRY, AMERICAN CHEMICAL SOCIETY. WASHINGTON, US, vol. 46, 2003, pages 1580-1588, XP002403225 ISSN: 0022-2623

## Description

This invention relates to pharmaceutical compositions. In particular, although not exclusively, it relates to formulation strategies for stabilising the pharmacologically-active ingredients of pharmaceutical compositions.

For many pharmacologically-active agents, the oral route of administration is preferred. However, in order for such agents to reach the bloodstream of the patient, they must usually be exposed to the contents of at least the upper part of the gastrointestinal tract (i.e. stomach and small intestine). Certain agents are sensitive to the acidic environment of the stomach. Such sensitivity usually results in acid-mediated hydrolysis of the agent. It is known to provide compositions containing such hydrolytically-sensitive agents with an enteric coating. Such a coating generally comprises an acidic polymer which is substantially uncharged and insoluble at low pH (i.e. in the stomach), but ionised and more soluble at higher pH values (i.e. on passage into the small intestine).

The compound 1A having the following structure: 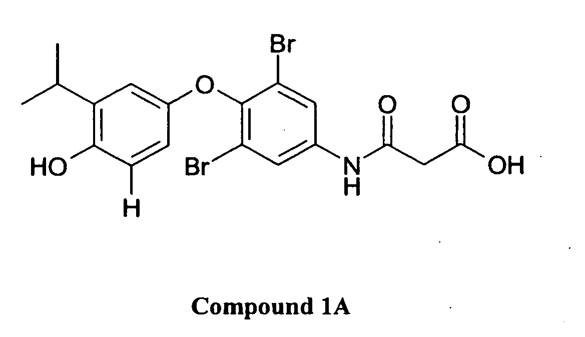 is described in WO 01/60784 (IUPAC name 3-[[3,5-Dibromo-4-[4-hydroxy-3-(1-methylethyl)-phenoxy]-phenyl]-amino]-3-oxopropanoic acid). Compound 1A and a series of related compounds are described as agonists of thyroid hormone receptors, in particular the TRβ receptor. Such compounds should be useful in the treatment or prevention of a disease associated with metabolic dysfunction or which is dependent upon the expression of a triiodothyronine (T₃)-regulated gene. Such diseases include, for example, obesity, hypercholesterolemia, atherosclerosis, cardiac arrhythmias, depression, osteoporosis, hypothyroidism, goitre, thyroid cancer as well as glaucoma and congestive heart failure.

In conducting formulation development work with compound 1A, the present inventors unexpectedly found that an undesirable transformation was taking place in the compound. A reaction product containing a nitro (-NO₂) group at the position *ortho* to the hydroxyl group on the phenolic ring (i.e. the left-hand ring as set out above) was being produced. On investigation, it was found that this reaction product had altered properties compared to the non-transformed compound 1 A, including, surprisingly, the potential for genotoxicity. Upon further examination, it was found that the reaction product was capable of being produced *in vivo* following oral administration. The present inventors therefore sought to investigate the process leading to the production of the nitrated reaction product with a view to inhibiting its formation following oral administration.

It is known (e.g. from Oldrieve et al., Chem. Res. Toxicol. 1998, 11, 1574), that certain flavonoid compounds are capable of inhibiting the nitration of tyrosine, or the formation of base deamination products from DNA bases, which occur under acidic conditions in the presence of nitrite. The nitration of hydroxyphenylacetic acid and proteins in the presence of nitrite and hydrogen peroxide in human saliva *in vitro* has been shown to be capable of inhibition by a reductant species (Takahama et al. Arch. Oral Biol. 2003, 48, 679). These disclosures do not allow the skilled person to predict, however, whether such nitration reactions may occur in other compounds, such as the structurally distinct thyroid hormone receptor agonists exemplified by compound 1A. In addition, they give no indication that such reactions may be of *in vivo* significance, and no suggestion as to predicting the potential properties (e.g. genotoxicity) of such nitrated reaction products.

The prior art does not disclose or suggest that thyroid hormone receptor-binding compounds such as compound 1 A above can be converted to potentially toxic reaction products on oral administration, nor how such a conversion may occur, nor how such a problem may be addressed.

It is therefore an object of the present invention to provide pharmaceutical compositions in which the above problem of nitration of certain thyroid hormone receptor-binding compounds following oral administration is attenuated.

Accordingly, a first aspect of the present invention provides a pharmaceutical composition suitable for oral administration, comprising:
(i) a compound of Formula I: 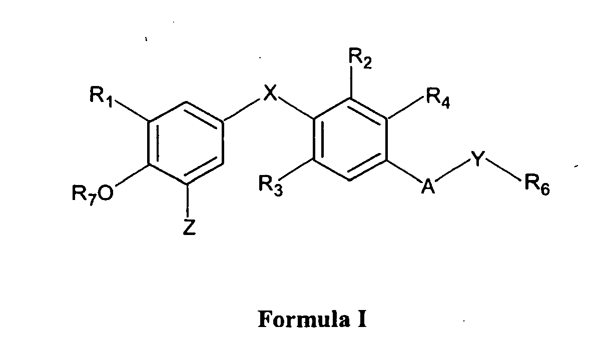 wherein:
   Z is H or an alternative group capable of being substituted by NO₂ via a nitrite-based nitration reaction;
   R₁ is selected from hydrogen, halogen, trifluoromethyl, or alkyl of 1 to 6 carbons or cycloalkyl of 3 to 7 carbons;
   X is oxygen (-O-), sulphur (-S-), carbonyl (-CO-), methylene (-CH₂-), or -NH-;
   R₂ and R₃ are the same or different and are hydrogen, halogen, alkyl of 1 to 4 carbons or cycloalkyl of 3 to 6 carbons, at least one of R₂ and R₃ being other than hydrogen;
   R₄ is hydrogen or lower alkyl;
   A is oxygen (-O-), methylene (-CH₂-), -CONR₅-, -NR₅-, or -NR₅CO-;
   R₅ is H or lower alkyl;
   R₆ is carboxylic acid (-CO₂H), or an ester thereof;
   Y is -(CH₂)ₙ, where n is 0, 1, 2, 3, 4 or 5 and wherein one or more of the CH₂ groups may optionally be substituted with halogen, or Y is -C=C-, which may be *cis* or *trans*; and
   R₇ is hydrogen, or an alkanoyl or aroyl group, or other group capable of bioconversion to generate the free phenol structure (wherein R₇ = H);
      including all stereoisomers thereof, or a pharmaceutically acceptable salt or ester thereof;
(ii) at least one pharmaceutically-acceptable excipient; and
(iii) an enteric coating.

Compounds of Formula I are particularly useful as thyroid hormone receptor agonists. In particular, many such compounds show enhanced activity at the TRβ (rather than TRα) receptor.

The composition of this first aspect of the invention is based on the surprising finding of the inventors, as a result of the investigations mentioned above, that the nitro reaction product of the compound 1A is formed upon oral administration via a nitrite-based nitration reaction. The nitrite-based nitration reaction requires a moderately low pH (around 2) in order to proceed. By providing the composition with an enteric coating (i.e. one which remains intact in the acidic stomach, and only dissolves on passage of the composition into the small intestine, where the pH is closer to neutral), the nitration reaction is significantly inhibited. The presence of the enteric coating prevents exposure of the pharmacologically-active compound of Formula I to the acidic media of the stomach, thereby preventing the nitrite reagent from being formed in the vicinity of the said active compound. The consequence of the enteric coating is therefore that the nitration reaction on oral administration of a compound of Formula I is attenuated. It should be emphasised that the motivation of the present inventors to understand and attempt to attenuate the nitration reaction was the unexpected production of a nitrated reaction product of compound 1A during formulation development work. Such a nitration reaction also occurs in other compounds of formula (I) including for example the compound GC-1 shown below. Unless this nitrated reaction product had been detected, there would have been no reason to determine its potential genotoxicity. Without knowledge of potential toxicity (or other undesirable characteristics) in such a reaction product, there would, of course, have been no incentive to attempt to prevent its formation.

The applicability of the present invention is not limited to the stabilisation of compound 1A. Rather, compositions in accordance with this first aspect of the invention should all experience a degree of stabilisation by virtue of attenuation of the nitration reaction which is liable to occur upon oral administration. The nitration of any compound of Formula I may lead to alterations in the compound's pharmacological and/or toxicological profiles, or equally may affect its pharmacokinetics. The nitro reaction products of such compounds may be potentially genotoxic. In certain cases, additional reactions in acidic media (e.g. liberation of aniline, which is itself potentially genotoxic), may also be prevented. In addition, nitration reactions in the non-prime (i.e. right-hand, as represented above) ring can also occur at low pH by means of electrophilic aromatic substitution. The enteric coating will also inhibit or prevent the formation of these nitro reaction products. In any case, the ability to control and/or prevent such chemical modifications of compounds of Formula I will generally be a useful tool for the formulator.

Compositions according to the first aspect of the invention allow for the compounds of Formula I only to be released from the composition once it has passed from the stomach into the intestine, at which point the enteric coating begins to dissolve and/or become permeable. Since the pH in the intestine is not low enough, however, for the nitrite-based nitration to occur to any significant extent, the nitration of the compounds of Formula I upon oral administration is significantly attenuated.

The term "alkanoyl" as employed herein alone or as part of another group is alkyl linked to a carbonyl group. The term "aroyl" as employed herein alone or as part of another group is aryl linked to a carbonyl group. Unless otherwise indicated, the term "alkyl" or "alk" as employed herein alone or as part of another group includes both straight and branched chain hydrocarbons, containing 1 to 12 carbons in the normal chain, preferably 1 to 4 carbons (in which case the term "lower alkyl" may be used), such as methyl, ethyl, propyl, isopropyl, butyl, t-butyl, or isobutyl, pentyl, hexyl, isohexyl, heptyl, 4,4-dimethylpentyl, octyl, 2,2,4-trimethylpentyl, nonyl, decyl, undecyl, dodecyl,. The term "aryl" as employed herein alone or as part of another group refers to monocyclic and bicyclic aromatic groups containing 6 to 10 carbons in the ring portion (such as phenyl or naphthyl including 1-naphthyl and 2-naphthyl) and may be optionally substituted through available carbon atoms with 1, 2, or 3 groups selected from hydrogen, halo, alkyl, haloalkyl, alkoxy, haloalkoxy, alkenyl, trifluoromethyl, trifluoromethoxy, alkynyl, hydroxy, amino, nitro, cyano and/or carboxyl or alkyl ester thereof. Unless otherwise indicated, the term "cycloalkyl" as employed herein alone or as part of another group includes saturated cyclic hydrocarbon groups or partially unsaturated (containing 1 or 2 double bonds) cyclic hydrocarbon groups, containing one ring and a total of 3 to 7 carbons, preferably 3 to 6 carbons, forming the ring, which includes cyclopropyl, cyclobutyl, cyclopentyl, cyclohexyl, cycloheptyl, cyclopentenyl and cyclohexenyl. The term "halogen" or "halo" as used herein alone or as part of another group refers to chlorine, bromine, fluorine, and iodine as well as CF₃, with chlorine or bromine being preferred.

The alternative group capable of being substituted by NO₂ via a nitrite-based nitration reaction may for example be selected from halo (e.g. iodo, chloro, bromo or fluoro, with the former preferred) and pseudohalogens (e.g. SCN, OCN, NCS, NCO and N₃).

The enteric coating is preferably formed using any commercially-available polymer produced for such a purpose. As examples of such polymers, those based on acrylates, methacrylates or copolymers thereof (such as the range of enteric coating polymers marketed under the name Eudragit® by Degussa/Roehm), polyvinyl acetate phthalate, cellulose acetate phthalate, hydroxypropylmethylcellulose acetate succinate, hydroxypropylmethylcellulose phthalate, hydroxymethylcellulose acetate succinate and carboxymethylethylcellulose may be mentioned. In a particular embodiment, the enteric coating comprises a methacrylic acid-ethyl acrylate copolymer. The constituent monomers of such a copolymer may be present in the ratio 1:1. The enteric coating also preferably contains a glidant component, such as talc. A plasticiser may also be advantageously included. A suitable plasticiser is triethyl citrate.

The enteric coated composition is preferably formulated such that 5% or less, more preferably substantially none, of the compound of Formula I is released in at least one hour, more preferably two hours, most preferably three hours, when release is measured in a USP dissolution apparatus II in either 900ml or 500ml of simulated gastric fluid or 0.1N HCl at 37°C with a stirring rate of 50 revolutions per minute. In preferred embodiments, at least 80%, preferably at least 90%, more preferably at least 95% and most preferably substantially all of the compound of Formula I is released, preferably within 1 hour, more preferably within 45 minutes, when measurement is carried out in pH 6.8 buffered medium (e.g. simulated intestinal fluid pH 6.8).

In some circumstances, it may be preferable for an inert coating to be provided between that portion of the composition containing the compound of Formula I, and the enteric coating (iii). Enteric coatings are typically composed of acidic polymers and hence, by their very nature, have the potential to lead to deleterious changes in certain active ingredients. An interposed inert coating (made from, for example, a cellulose derivative, such as hydroxypropyl cellulose or hydroxypropylmethyl cellulose) tends to inhibit such interactions. The inert coat should, of course, be soluble (or otherwise dispersible) in the intestinal media in order to allow the compound of Formula I to be released.

The term 'enteric coating' as used herein is intended to include coatings applied to a composition/dosage form (e.g. a tablet) once the dosage form is otherwise essentially complete and those applied at intermediate stages of dosage form manufacture. Thus, compositions are included in which the compound of Formula I is formulated with excipients into granules which are then enteric coated prior to further processing, such as compression into tablets or filling into capsules, for example gelatin capsules. Equally, an approach such as that described in WO 00/22909, whilst less preferred, may be suitable for preparing compositions according to the first aspect of the invention for certain compounds of Formula I. In this approach, complexes between pharmacologically-active ingredients and relatively hydrophobic carboxylic acids (e.g. C₉ to C₃₀ aliphatic carboxylic acids) are prepared by co-precipitation from solution by adjustment of pH.

In certain compounds of Formula I, X is oxygen or -CH₂-. Alternatively or in addition, A may be -NH-, -O-, -CH₂- or -CONR₅-. In some preferred compounds of Formula I, R₁ is isopropyl, iodo or H. In addition or alternatively, it may be preferred that R₂ and R₃ are each independently halogen or alkyl. In such a case, R₂ and R₃ are preferably each independently Cl, Br, I or methyl. In certain preferred embodiments, R₂ = R₃. R₂ = R₃ = Br or Cl are especially preferred.

In compounds of Formula I, R₄ is preferably H or methyl, with H particularly preferred.

In certain compounds of Formula I, Y is -(CH₂)ₙ- and n is 1 or 2. Alternatively or in addition, A may be -NR₅CO-, with R₅ being H. In particularly preferred compounds of Formula I, Y is -(CH₂)ₙ- with n=1, A is NHCO or CONH and R₆ is COOH, or a corresponding salt or ester of COOH.

The compound (i) may, in selected embodiments of the present invention, have a formula selected from the following: 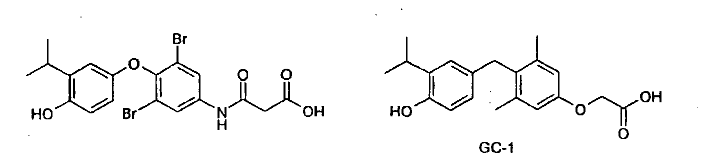 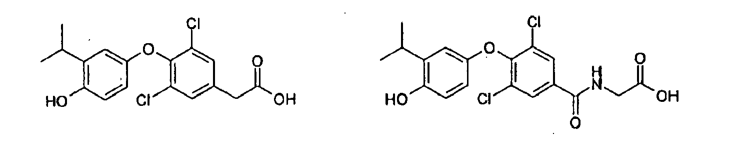 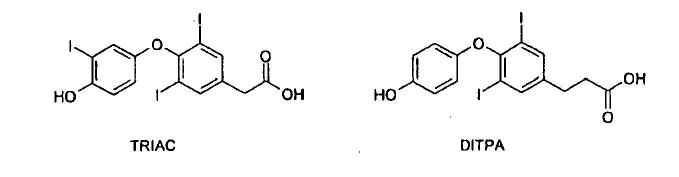 or a pharmaceutically acceptable salt or ester thereof.

As would be understood by the skilled person, the nitration reaction described above would lead to the introduction of a nitro group at the lowermost position of the left-hand benzene ring as represented in the above structures, or *ortho* to the OH. Accordingly, whilst in most cases the substituted group would be H, alternative groups capable of being substituted by means of a nitrite-based nitration reaction are also contemplated, e.g. an iodo group *ortho* to the phenol hydroxyl group. The inventors have shown this to be a facile reaction, albeit slower than the replacement of hydrogen.

When a compound of Formula I is present in the form of an ester, an alkyl ester thereof is preferred. When a compound of Formula I is present in the form of a pharmaceutically acceptable salt, such salts may include, when the compound has at least one basic centre, acid addition salts, e.g. with strong inorganic acids, such as mineral acids, for example sulfuric acid, phosphoric acid or a hydrohalic acid, with strong organic carboxylic acids, such as alkanecarboxylic acids of 1 to 4 carbon atoms which are unsubstituted or substituted, for example, by halogen, for example acetic acid, such as saturated or unsaturated dicarboxylic acids, for example oxalic, malonic, succinic, maleic, fumaric, phthalic or terephthalic acid, such as hydroxycarboxylic acids, for example ascorbic, glycolic, lactic, malic, tartaric or citric acid, such as amino acids, (for example aspartic or glutamic acid or lysine or arginine), or benzoic acid, or with organic sulfonic acids, such as (C1-C4) alkyl or arylsulfonic acids which are unsubstituted or substituted, for example by halogen, for example methanesulfonic acid or p-toluenesulfonic acid.

Corresponding acid addition salts can also be formed having, if desired, an additionally present basic centre.

When the compound of Formula I has at least one acid group (for example COOH), it can also form salts with bases. Suitable salts with bases are, for example, metal salts, such as alkali metal or alkaline earth metal salts, for example sodium, potassium or magnesium salts, or salts with ammonia or an organic amine, such as morpholine, thiomorpholine, piperidine, pyrrolidine, a mono, di or tri-lower alkylamine, for example ethyl, tertbutyl, diethyl, diisopropyl, triethyl, tributyl or dimethylpropylamine, or a mono, di or trihydroxy lower alkylamine, for example mono, di or triethanolamine. Corresponding internal salts may furthermore be formed.

Preferred salts of the compounds of Formula I which include a basic group include monohydrochloride, hydrogensulfate, methanesulfonate, phosphate or nitrate. Preferred salts of the compounds of Formula I which include an acid group include sodium, potassium and magnesium salts and pharmaceutically acceptable organic amines.

The compounds (i) may of course be solvated if desired, for example hydrates may be used in the present invention.

In certain embodiments of the first aspect of the invention, the composition also includes an antioxidant. Such embodiments are based on the unexpected finding of the inventors, as a result of the investigations mentioned above, that the nitro reaction product of the compound 1A is formed upon oral administration via a nitrite-based reaction which is free radical-mediated. It has also been determined by the inventors that the non-biaryl ether compound GC-1, also described above, becomes nitrated readily under physiologically relevant conditions. The incorporation of the antioxidant into the composition of the first aspect of the invention inhibits the formation of the free radicals and/or scavenges free radicals which are formed, with the consequence that any nitration reaction which occurs in spite of the enteric coating is attenuated.

The antioxidant is preferably water soluble. Such antioxidants include ascorbic acid, fumaric acid, malic acid, propionic acid, or a salt of any of the said acids, monothioglycerol, potassium metabisulphite, sodium bisulphite, sodium sulphite and sodium metabisulphite. A preferred antioxidant is ascorbic acid or its sodium salt, which has been found to have particularly significant inhibitory effects on the nitration reaction.

Alternatively, the antioxidant may be water insoluble. Such an antioxidant may be selected from α-tocopherol, ascorbyl palmitate, butylated hydroxyanisole, butylated hydroxytoluene, ethyl oleate and propyl gallate.

Although not essential, it may be preferable for the compound (i) and the antioxidant (ii) to be substantially homogeneously mixed. This helps to ensure that, upon any contact of the compound (i) with nitrite (and/or nitrous acid, and/or any nitrogen- and oxygen-containing free radical species), the antioxidant is more likely to be in the vicinity of such contact and thus able to better attenuate any nitration reaction which may occur.

In accordance with a second aspect of the present invention, there is provided a pharmaceutical composition suitable for oral administration, comprising:
(i) a compound of Formula I or a pharmaceutically acceptable salt or ester thereof, as described above;
(ii) at least one antioxidant; and
(iii) at least one pharmaceutically-acceptable excipient.

In preferred embodiments of the second aspect of the invention, the composition is a solid composition. The preferred features of Formula I as described in relation to the first aspect of the invention also apply in connection with the second aspect (and those other aspects described below, as appropriate).

The composition of the second aspect of the invention preferably also includes an enteric coating. The enteric coating may be as described above in relation to the first aspect of the invention.

The present invention furthermore provides, in a third aspect, a method of stabilising a pharmaceutical composition suitable for oral administration, the pharmaceutical composition comprising:
(i) a compound of Formula I or a pharmaceutically acceptable salt or ester thereof as described above; and
(ii) at least one pharmaceutically-acceptable excipient;
the method comprising providing the composition with an enteric coating.

In a further aspect, the invention provides, in a pharmaceutical composition suitable for oral administration containing a compound of Formula I or a pharmaceutically acceptable salt or ester thereof as defined above, the use of an enteric coating or an antioxidant or both an enteric coating and an antioxidant for reduction or prevention of nitration of said compound.

The present invention also provides a composition according to the invention, for use in therapy.

The present invention also provides the use of a composition according to the invention in the preparation of a medicament for preventing, inhibiting or treating a disease selected from obesity, hypercholesterolemia, dyslipidaemia, atherosclerosis, cardiac arrhythmias, depression, osteoporosis, hypothyroidism, goitre, thyroid cancer as well as glaucoma and congestive heart failure.

The invention finds utility in a method of preventing, inhibiting or treating a disease associated with metabolism dysfunction, or which is dependent on the expression of a triiodothyronine (T₃)-regulated gene, the method involving the administration of a composition according to the invention to a subject in need of such prevention, inhibition or treatment.

In the use and method described immediately above, the medicament or composition may be administered at a dosing interval of from 30 minutes to one month. More preferably, the dosing interval is from one to seven days, even more preferably one to three days. The typical adult human dose range for compounds (i) would be around 1µg to around 2000 µg per day. For many compounds (i), the daily dose would be less than 300 µg. Preferably, the dose of compound (i) is from around 1 µg to around 200 µg per day, more preferably around 1 to around 100 µg per day. For example, the compounds (i) may be administered in one dose, two doses, three doses or four doses per day. Preferably, the amount of compound (i) per unit dose of composition is from 1 to 200 µg, more preferably 1 to 100 µg, more preferably 1, 5, 10, 20, 25 or 50 µg. For example, the amount of compound (i) per unit dose may be from 10 to 100, for example from 20 to 80, typically from 25 to 50, µg.

The composition according to the first or second aspect of the invention may also comprise a further pharmacologically active ingredient selected from hypolipidaemic agents, antidiabetic agents, antidepressants, bone resorption inhibitors, appetite suppressants and/or anti-obesity agents.

The further pharmacologically active ingredients tend to have additive or synergistic effects with the compounds (i) so as to enhance the metabolic effects thereof.

In compositions as described above containing an antioxidant, the amount of antioxidant may vary over a very wide range. Preferably at least 0.0001 mmol of antioxidant are present, for example 0.0005 mmol, more preferably at least 0.01 mmol. The amount of antioxidant may for example be from 0.0001 to 15 mmol, for example 0.0005 to 10 mmol, typically 0.01 to 4 mmol per dose of composition.

As will be described in more detail below, the average human swallows around 0.1 mmol of salivary nitrite per hour. The presence of the above quantities of antioxidant in the composition should provide a useful stabilising effect for the compound (i), even if the composition of the invention is resident in the stomach for a relatively protracted period.

In yet another aspect, the present invention provides a combination medicament suitable for oral administration, comprising:
(1) a first pharmaceutical composition comprising a compound of Formula I or a pharmaceutically acceptable salt or ester thereof, as described above; and
(2) a second pharmaceutical composition comprising at least one antioxidant,
wherein each of the first and second pharmaceutical compositions contains at least one pharmaceutically-acceptable excipient, and wherein the first and second pharmaceutical compositions may be administered simultaneously, sequentially or separately.

The combination medicament of the present invention takes advantage of the fact that, in order for the stabilisation of the compound (i) to be effected, the antioxidant should merely be present when the former comes into contact with acid and a source of nitrite. Provided the two compositions of the combination medicament are given in such temporal proximity that there is overlap between their periods of residence in the stomach, the compound (i) should enjoy at least a degree of stabilisation against the nitration reaction. The compositions, or at least the first composition, are preferably solid compositions.

The invention will now be described in more detail by way of example only and with reference to the appended drawings, of which:
Figure 1 which shows a series of standard HPLC traces of the compound 1A in the presence of various concentrations of its 5'-nitro reaction product; and
Figure 2 illustrates a process for manufacturing enteric coated tablets containing compound 1A.

As mentioned above, during development of compound 1A, it was determined that the formation of the 5'-nitro reaction product was taking place through an abiotic (i.e. non-enzymatic) route. The toxicological properties of the nitro reaction product were determined to be altered to a relevant extent as compared to the parent compound. It was therefore important to determine how the nitration reaction was occurring and to devise approaches to control or prevent this *in vivo.*

### Example 1 - Genotoxicity of a reaction product of Compound 1A

### 1.1 Introduction

During development of a 25 µg capsule formulation of compound 1A, a previously undetected impurity was observed in several batches; the impurity ranged from 0.8 to 1.2 % of parent compound. This impurity was identified as a nitro analog, hereinafter referred to as reaction product 1B.

A theoretical potential for the *in vivo* formation of this impurity/reaction product was therefore identified. Hence, this putative reaction product was tested for genotoxic activity in an International Conference on Harmonisation (ICH) standard battery of studies.

This example summarizes the genotoxicity studies conducted with reaction product 1B and relates the findings to clinical exposures to this impurity/reaction product.

### 1.2 Genotoxicity

### 1.2.1 Reaction product 1B

### 1.2.1.1 Exploratory Ames assay

Reaction product 1B was tested in duplicate cultures in the presence and absence of S-9 metabolic activation. The positive-control articles were tested in duplicate cultures and prepared in DMSO, with the exception of sodium azide, which was dissolved in water. The negative (vehicle) controls were tested in replicates of five cultures.

Reaction product 1B exhibited cytotoxicity to each of the S. *typhimurium* and *E. coli* strains tested. Cytotoxicity ranging from minimal to marked was apparent based on reductions in mean revertant number and/or reductions in the bacterial-background lawn density. When compared to the negative controls, the histidine⁺ revertant values were elevated in the reaction product 1B-treated cultures of strain TA100 in the presence and absence of S-9 activation, respectively. As expected, significant increases in the histidine⁺ and tryptophan⁺ revertant numbers were observed in the cultures treated with the positive-control compounds.

In conclusion, reaction product 1B showed a positive response in this study.

### 1.2.1.2 Cytogenetic study in primary human lymphocytes

An *in vitro* cytogenetics study was performed to investigate the potential of reaction product 1B to induce chromosome aberrations in cultured human lymphocytes. Based on range-finding cytotoxicity results, concentrations of 5 to 30 µg/ml were selected for evaluation in the 24-hr exposure without metabolic enzyme activation and concentrations of 2.5 to 20 µg/ml for the 5-hr exposure with Aroclor 1254-induced rat liver (S9 fraction) metabolic activation.

In the 5-hr exposure to reaction product 1B in the presence of S-9 metabolic activation, a statistically significant increase in the frequency of chromosome aberrations occurred. At the highest concentration, 20 µg/ml, the frequency of chromosome aberrations was 10.5 % compared to 2.5 % for the vehicle control and the reduction in the mitotic index was approximately 51 %.

In the 24-hr exposure to reaction product 1B in the absence of S-9 metabolic activation, a statistically significant increase in the frequency of chromosome aberrations occurred. At the highest concentrations evaluated, 40 µg/ml, the frequency of chromosome aberrations was
7.5 % compared to 2.5 % for the vehicle control with a reduction in the mitotic index of approximately 48 %.

As expected, the positive controls in each trial induced statistically significant increases in the frequencies of damaged metaphases. Thus, the validity of this assay was demonstrated.

In conclusion, reaction product 1B was clastogenic to dividing human lymphocytes when tested to the maximum concentrations recommended by international guidelines for *in vitro* cytogenetic studies.

### 1.2.1.3 Oral micronucleus study in mice

Reaction product 1B was evaluated in the mouse bone-marrow micronucleus assay to determine its *in vivo* genotoxic potential. Groups of mice were given three consecutive daily oral doses of 1000, 1500, or 2000 mg/kg of reaction product 1B (i.e. up to the maximum dose level required by international regulatory guidelines in ICH and OECD). Femur bone-marrow samples were collected from all animals approximately 24 hr following the last dose for evaluation of micronucleated polychromatic erythrocytes (MN-PCE).

No animals died during the study and no drug-related clinical signs were observed. No bone-marrow toxicity was observed as measured by meaningful decreases in polychromatic erythrocytes (PCE). The frequencies of MN-PCE were statistically increased at 1000 and 1500 mg/kg.

In conclusion, non dose-dependant positive responses were found in mice.

### 1.3 Clinical Exposure to Reaction Product 1B

Following administration of single oral doses of compound 1A to human volunteers, reaction product 1B could be detected in plasma. Similarly, in a trial in which subjects received daily doses of compound 1A for two weeks, reaction product 1B could be detected in several subjects.

In conclusion, following dosing of compound 1A in humans, the potentially mutagenic nitro reaction product 1B could be detected in plasma. This is the case even if dosing is carried out over a relatively short period (e.g 14 days, as above). Potential genotoxins are typically non-dose-dependent in their mutagenic effects and hence the presence of even low levels of the reaction product 1 B are of clinical relevance. In addition, compounds of Formula I would typically be given over a long period of time and hence the prevention of the nitro reaction products being formed is of paramount importance if potential genotoxic effects resulting from accumulation of the reaction products are to be avoided.

### 1.4 Summary and Conclusion

The impurity/reaction product of compound 1A, reaction product 1B, was found to induce chromosomal aberrations in human peripheral lymphocytes *in vitro* and non dose-dependant micronuclei in a mouse micronucleus study *in vivo*.

Furthermore, the reaction product 1B could be detected in human plasma following short-term dosing of compound 1A. Accordingly, it is clear that the prevention of formation of the nitro reaction product should be of significant benefit, both because potential genotoxins can exert genotoxic effects at very low concentrations and because compounds of Formula I, such as compound 1A, will generally be given chronically.

### Example 2 - Nitrate and Nitrite - Sources and nitration mechanisms

### 2.1 Introduction

A possible source of nitration is the non-classical nitrite pathway. This pathway was first reported by Uemura et al (1978, J. Chem. Soc. Perkin Trans. I, 9, 1076). The nitrite pathway can proceed at moderately low pH (around 2) and is tolerant of water, in contrast to the nitrate pathway. The nitrite pathway leads to the production of free radical species capable of reacting with hydroxylated benzene-containing compounds, (Beake et al. (1992) J. Chem. Soc. Perkin Trans. 2, 10, 1653):

The present inventors considered that this pathway was likely to be the main pathway for nitration of orally-administered compounds of Formula I in the stomach and intestine.

The average daily intake of nitrate and nitrite from sources other than food additives has been estimated.(T. Hambridge, WHO Food Additives Series: 50, Nitrate and Nitrite).

As far as endogenous sources of nitrite are concerned, it is known that nitrate is produced from the nitrate in saliva by the action of oral nitrate reductase (Benjamin (2000) Ann. Zootech. 49, 207). This results in a concentration of around 200µM nitrite in the saliva. The average human swallows around 500ml of saliva per hour. This results in the ingestion of around 2.4mmol (110 mg) of nitrite per day. This amounts to about 1.6mg/kg/day nitrite (for an average adult weighing 70kg).

### 2.2. In vivo nitration study

In experiments conducted *in vivo* in rats, it was found that, on administration of compound 1A together with a nitrate and nitrite solution (54mg and 4mg, respectively, per rat), around 6% of the administered dose of compound 1A was nitrated.

### 2.3. Antioxidants

As set out above, it was hypothesised that the mechanism of nitration of compound 1A (and hence other hydroxylated benzene-containing compounds, such as those of Formula I) was via nitrite and hence free radical-mediated. Accordingly, it was considered that the nitration reaction could be inhibited by free radical scavengers/antioxidants.

In general, any antioxidant can be used in accordance with the present invention. 'True' antioxidants are those which block radical-mediated chain reactions by reacting with the free radicals (by donating a single electron to the radical species). An example of such a true antioxidant is butylated hydroxytoluene (BHT). Other examples of such species include the phenolic antioxidants, such as ferulic acid, rutin, catechin, epicatechin, epigallocatechin, apicatechingallate and epigallocatechingallate. Many such species are naturally-occurring, e.g. flavonoid- or *trans*-stilbene-type antioxidants. Reducing agents are species having a lower redox potential than the compound they are being employed to protect, and/or they may act as nitration decoys. An example of an agent acting in this way is ascorbic acid. In addition, certain agents are 'antioxidant synergists'. These agents enhance the effects of antioxidants and may also be included in compositions of the present invention; an example is sodium edetate.

Antioxidants can also be grouped according to their solubility characteristics. Water soluble antioxidants include ascorbic acid (or its sodium salt), fumaric acid, malic acid, monothioglycerol, potassium metabisulphite (KO₃S-SO₂K), propionic acid (CH₃CH₂CO₂H), sodium bisulphite (NaHSO₃) and sodium sulphite (Na₂SO₃). Water insoluble antioxidants include alpha tocopherol, ascorbyl palmitate. butylated hydroxyanisole (BHA), butylated hydroxytoluene (BHT), ethyl oleate and propyl gallate.

### 2.4. The nitration reaction and its inhibition

It is known that L-tyrosine can be converted to a nitro reaction product in which the NO₂ group is substituted *ortho* to the ring hydroxyl group. The experimental procedure for determining the extent of nitration of L-tyrosine was used for determining the nitration of compound 1A and the inhibition thereof by selected antioxidants. The experimental procedure for L-tyrosine is known (1998, Chem. Res. Toxicol., 11, 1578). In brief, solutions of compound 1 A (50µM) were exposed to solutions of NaNO₃ (2500µM) and/or NaNO₂ (various concentrations) at various pH values, at 37°C and in the presence or absence of phosphate buffer. The nitration reaction leads to the formation of the 5'-nitro reaction product of compound 1A. This reaction product can be detected and quantified using HPLC, the reaction product having a reduced retention time (under the conditions used) compared to the parent compound. Antioxidant inhibitors were then added to NaNO₂- and 1 A-containing test solutions and the nitration tests repeated.

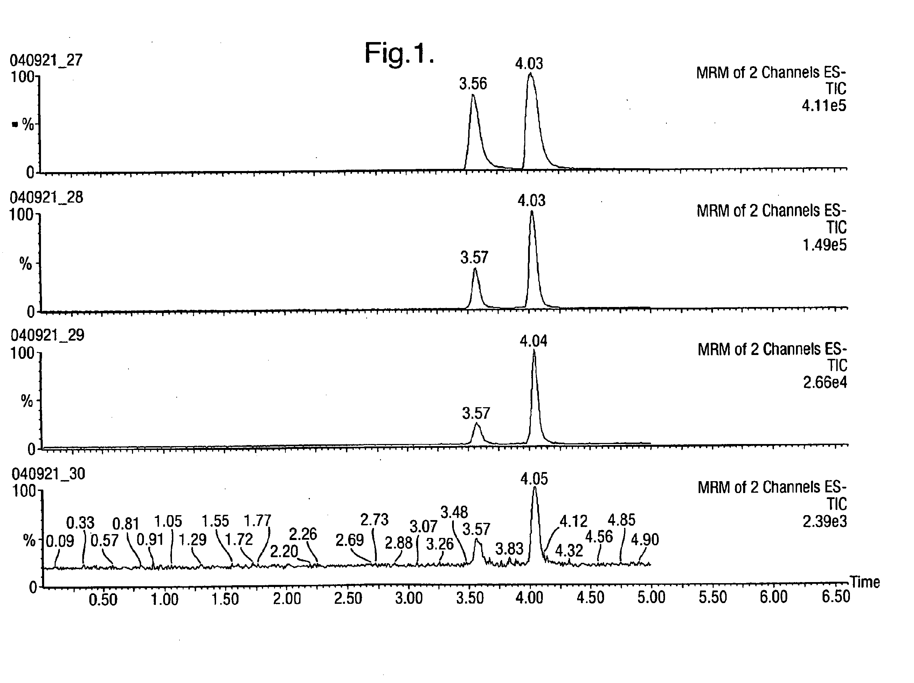

Figure 1 shows a series of standard HPLC chromatograms of 1A (50µM) in the presence of 50nM to 50µM reaction product 1B. Analysis was by HPLC-MS/MS, with the following conditions:

### HPLC Conditions

Instrument: Waters Alliance 2795 LC
Guard Column: Waters Sentry Guard Column, Symmetry C₁₈ 3.5µm, 2.1 x 10mm.
Column: Waters Symmetry C₁₈ 3.5µm, 2.1 x 50 mm.
Column Temperature: 45 °C
Injection Volume: 5µl
Autosampler Temperature: 10°C
Flow Rate: 0.2 ml/min
Mobile Phase:
A= 10 mM Ammonium acetate (pH=4.5 with acetic acid),
B= Acetonitrile

Gradient:

| **Time (min)** | **% A** | **% B** | **Flow Rate (ml/min)** | **Curve** |
|---|---|---|---|---|
| 0.00 | 90 | 10 | 0.2 | linear |
| 1.00 | 0 | 100 | 0.2 | linear |
| 2.00 | 0 | 100 | 0.2 | linear |
| 2.10 | 90 | 10 | 0.2 | linear |
| 4.00 | 90 | 10 | 0.2 | linear |

### Mass Spectrometry Conditions:

Instrument: Micromass Quattro micro API
Mode: ESI -
Experiment: MRM (multiple reaction monitoring)
MRM transitions for compound 1A: m/z 486.0 > 399.9 (t_{R}=3.47 min)
MRM transitions for reaction product 1B: m/z 531.0 > 444.8 (t_{R}=3.91 min)

### Limit of Quantification (LOQ):

LOQ for compound 1A in MRM is 10nM in 50%acetonitrile:50% water.
LOQ for reaction product 1B in MRM is 1nM in 50%acetonitrile:50% water.

It can clearly be seen that the reaction product has a reduced retention time under the conditions used, with the parent compound 1A eluting at around 3.5 minutes and the reaction product eluting at around 4 minutes.

On incubation of 1A with various concentrations of NaNO₂ and/or NaNO₃, the results of Table 1 were obtained.

**Table 1: Nitration of compound 1A in the presence of nitrite and/or nitrate and under various conditions of pH.**

| **1A** | **NaNO₃** | **NaNO₂** | **Inhibiting** | | **Time** | **Nitration** |
|---|---|---|---|---|---|---|
| **(µM)** | **(µM)** | **(µM)** | **Agent** | **pH** | **(hr)** | **(%)** |
| 50 | | | | 2 (HCl) | 24 | 0 |
| 50 | | | | 2 (phosphate) | 24 | 0 |
| 50 | | | | 6.4 | 24 | 0 |
| 50 | | | | 7.2 | 24 | 0 |
| 50 | **2500** | **100** | | 2 (HCl) | 4 | **8.4** |
| 50 | 2500 | 100 | | 2 (phosphate) | 4 | 9.63 |
| 50 | 2500 | 100 | | 6.4 | 4 | 0.017 |
| 50 | 2500 | 100 | | 7.2 | 4 | 0 |
| 50 | 2500 | | | 2 (HCl) | 4 | 0 |
| 50 | 2500 | | | 2 (phosphate) | 4 | 0 |
| 50 | 2500 | | | 6.4 | 4 | 0 |
| 50 | 2500 | | | 7.2 | 4 | 0 |
| 50 | | **100** | | 2 (HCl) | 4 | **98.6** |
| 50 | | 100 | | 2 (phosphate) | 4 | 4.27 |
| 50 | | 100 | | 6.4 | 4 | 0.013 |
| 50 | | 100 | | 7.2 | 4 | 0 |

Several conclusions can be made on the basis of these data. First, it is clearly demonstrated that nitrite alone, under appropriate conditions of acidity, is sufficient to cause significant nitration of the compound 1A. As shown by the negative control (rows 1 to 4), no nitration result is observed when nitrite and nitrate are absent. Second, the presence of the phosphate buffer may actually lead to a significant inhibition of the nitrite-based reaction, the reaction occurring to a much greater extent in pH 2 HCl solution than in pH 2 buffer. Third, nitrate at pH 2 (HCl or buffer) is not capable of leading to measurable nitration of compound 1A. Fourth, nitrate appears to inhibit the nitrite-based reaction to a significant extent. (more than one order of magnitude; see figures in bold type).

These data thus confirm that the nitration reaction observed with hydroxylated benzene-containing compounds, such as those of Formula I (e.g. compound 1A), when administered orally, is mediated by nitrite rather than nitrate. The data also clearly demonstrate the importance of an acidic environment for the nitration reaction. The enteric coated compositions of the present invention reduce or prevent access of acidic media to the pharmacologically active ingredients contained therein. Accordingly, such a formulation approach is capable of significantly reducing the nitration reaction which would otherwise occur.

On addition of selected antioxidant inhibiting agents to compound 1A in the presence of nitrite, the results of Table 2 were achieved. The amount of antioxidant used was generally around 10-20 times the amount of compound 1A.

**Table 2: Inhibition of nitration of compound 1A in the presence of nitrite by means of antioxidant inhibitor agents.**

| **1A(µM)** | **NaNO₂(µM)** | **Inhibiting Agent** | **pH** | **Time(hr)** | **Nitration(%)** |
|---|---|---|---|---|---|
| 50 | 100 | | 2 (HCl) | 4 | 98.6 |
| 50 | 100 | | 6.4 | 24 | 0.02 |
| 50 | 400 | | 6.4 | 24 | 0.12 |
| 50 | 800 | | 6.4 | 24 | 0.21 |
| 50 | 100 | BHT | 2 (HCl) | 24 | 0.02 |
| 50 | 100 | Sodium ascorbate | 2 (HCl) | 4 | 0.00 |
| 50 | 100 | Sodium ascorbate | 2 (phosphate) | 4 | 0.00 |
| 50 | 100 | Sodium ascorbate | 6.4 | 4 | 0.00 |
| 50 | 100 | Sodium ascorbate | 7.2 | 4 | 0.00 |
| 50 | 100 | Sodium bisulfite | 2 (HCl) | 0 | 0.77 |
| 50 | 100 | Sodium bisulfite | 2 (phosphate) | 0 | 0.11 |
| 50 | 100 | Sodium bisulfite | 2 (HCl) | 2 | 0.11 |
| 50 | 100 | Sodium bisulfite | 2 (phosphate) | 2 | 0.16 |
| 50 | 100 | Sodium bisulfite | 6.4 (NaAc) | 2 | 0 |
| 50 | 100 | Sodium bisulfite | 6.4(phosphate) | 2 | 0 |
| 50 | 100 | Sodium bisulfite | 2 (HCl) | 2 | 0.11 |
| 50 | 100 | Sodium bisulfite | 2 (phosphate) | 4 | 0.17 |
| 50 | 100 | Sodium bisulfite | 6.4 (NaAc) | 4 | 0 |
| 50 | 100 | Sodium bisulfite | 6.4(phosphate) | 4 | 0 |
| 50 | 100 | Catechin | 2 (HCl) | 4 | 0.01 |
| 50 | 100 | Catechin | 2 (phosphate) | 4 | 0.01 |
| 50 | 100 | Catechin | 6.4(NaAc) | 4 | 0 |
| 50 | 100 | Catechin | 6.4(phosphate) | 4 | 0 |
| 50 | 100 | Catechin | 2 (HCl) | 24 | 0.02 |
| 50 | 100 | Catechin | 2(phosphate) | 24 | 0 |
| 50 | 100 | Catechin | 6.4(NaAc) | 24 | 0 |
| 50 | 100 | Catechin | 6.4(phosphate) | 24 | 0 |

The data of Table 2 allows a number of significant conclusions to be drawn. It is clear that, unless the nitrite is acidified, no or insignificant nitration of the compound 1A takes place, even when the nitrite concentration is increased up to eight times. More importantly, it is seen that, under conditions of pH and nitrite concentration which are found to lead to an almost total conversion of the test compound to its nitro reaction product, the presence of BHT (water insoluble) or ascorbate (water soluble) leads to an essentially complete inhibition of the nitrite-mediated nitration (see figures in bold type).

Overall, therefore, the data reported herein clearly illustrate that nitrite (NO₂) and not nitrate (NO₃) is the likely source of the *in vivo* nitration observed in hydroxylated benzene-containing compounds, such as those of Formula I (e.g. compound 1A). This nitrite-mediated nitration proceeds via a free radical mechanism and only takes place to a relevant extent in acidified media. The incorporation of an antioxidant into a composition containing the hydroxylated benzene-containing compound inhibits this nitration reaction to a significant extent. In addition or alternatively, the protection of the composition by means of an enteric coating will prevent acidified nitrite from coming into contact with the hydroxylated benzene-containing compound and thus provides a potent strategy for stabilising the compound against nitration.

### Example 3 - An enteric coated formulation of Compound 1A

### 3.1 Particle size measurements of Compound 1A before and after milling.

As the unmilled compound 1A contained a large proportion of particles larger than approximately 100 µm, which will affect the content uniformity of a tablet, the compound 1A was milled. A Retsch MM 2000 was used for milling 2 x 2 g compound 1A. A spherical particle of 100 µm diameter with a density of 1.5 g/cm³ has a mass of approx. 1 µg.

The particle size distribution was measured with a Malvern MasterSizer 2000, i.e. laser diffraction technique. The powder sample is dispersed in Tween 20 and water.

*Unmilled Compound 1A:* Measurements revealed median diameters, d(0.5), of 101 and 103 µm. The 90 % quartiles, d(0.9), were 158 and 159 µm.

*Milled Compound 1A:* After milling d(0.5) was 20 µm and d(0.9) was 85 µm. This was an acceptable particle size distribution.

### 3.2 Tablet formula

The medicinal product made was a white, circular (diameter 7mm), convex, enteric film-coated tablet of two strengths, 50 and 300 µg of compound 1A (hereinafter '1A') per tablet. The complete composition of the medicinal product is provided in Table 3.1, below.

**Table 3.1: Composition of 1A enteric coated tablets**

| **Ingredient** | **Quantity, mg/unit** | **Quantity, mg/unit** | **Standard** |
|---|---|---|---|
| 1A | 0.050 | 0.300 | |
| Mannitol | 54.6 | 54.6 | Ph.Eur. |
| Cellulose, microcrystalline | 83.65 | 83.4 | Ph.Eur. |
| Hypromellose | 0.3 | 0.3 | Ph.Eur. |
| Magnesium stearate | 1.4 | 1.4 | Ph.Eur. |
| Water, purified* | 15 | 15 | Ph.Eur. |
| Methacrylic acid-ethyl acrylate copolymer (1:1) dispersion 30 % | 25.0 (7.5**) | 25.0 (7.5**) | Ph.Eur. |
| Talc | 3.75 | 3.75 | Ph.Eur. |
| Triethyl citrate | 0.75 | 0.75 | Ph.Eur. |
| Water, purified* | 50.5 | 50.5 | Ph.Eur. |

| | | | |
|---|---|---|---|
| * *Evaporates during the manufacturing process* *** Quantity of dry copolymer within parenthesis* | | | |

The target weight of the core tablets was 140 mg and the target weight of the film was 12 mg.

### Batch Formula

Batch formula (see Table 3.2) for 50 µg/tablet and 300 µg/tablet refers to 6000 pcs (840 g) of core tablets during tablet production and 5700 pcs (798 g) of core tablets during coating.

**Table 3.2: Batch Formula of 1A enteric coated tablets**

| **Ingredient** | **Amount per batch (50 µg/tablet), g** | **Amount per batch, (300 µg/tablet), g** | **Standard** |
|---|---|---|---|
| 1A | 0.318* | 1.908* | |
| Mannitol | 327.6 | 327.6 | Ph.Eur. |
| Cellulose, microcrystalline | 501.9 | 500.4 | Ph.Eur. |
| Hypromellose | 1.8 | 1.8 | Ph.Eur. |
| Magnesium stearate | 8.4 | 8.4 | Ph.Eur. |
| Water, purified** | 90 | 90 | Ph.Eur. |
| Methacrylic acid-ethyl acrylate copolymer (1:1) dispersion 30 per cent | 158.2 (47.4***) | 158.2 (47.4***) | Ph.Eur. |
| Talc | 23.7 | 23.7 | Ph.Eur. |
| Triethyl citrate | 4.7 | 4.7 | Ph.Eur. |
| Water, purified** | 320 | 320 | Ph.Eur. |

| | | | |
|---|---|---|---|
| ** Includes 6% overage due to losses during the production of the core tablets* *** Evaporates during the manufacturing process* **** Quantity of dry copolymer within parenthesis* *The amount of coating suspension (the last four rows of the table) includes an overage of 11 % due to losses during the coating process.* | | | |

### 3.3 Manufacture

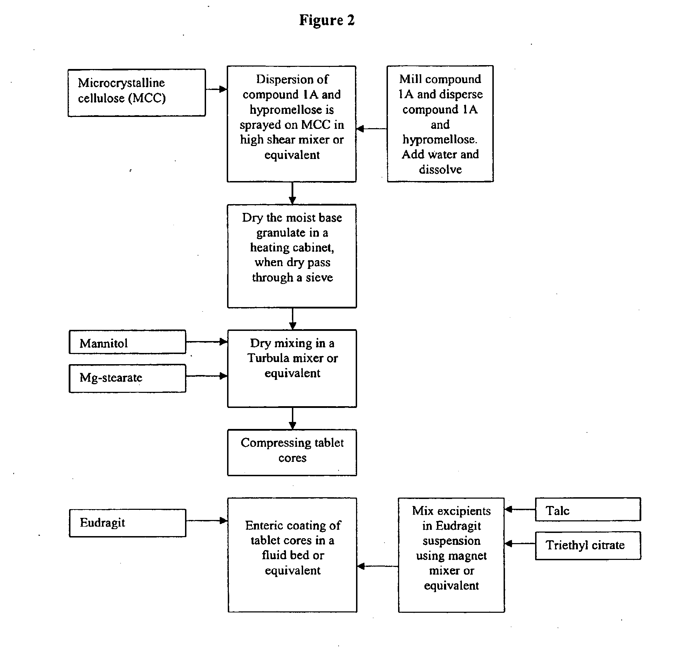

A flow diagram of the manufacturing process of 1 A Enteric-coated Tablets is given in Figure 2. Core tablets with 50 µg and 300 µg 1A were made. The core tablets are a mixture of a "base granulate" (containing 1A), mannitol, hypromellose and magnesium stearate. The "base granulate" is made by suspending milled 1A in an aqueous solution of hypromellose and spraying the dispersion on MCC. Six percent overage of 1 A is used due to losses. After evaporating the water, the dried product is sieved. The powder mixture is compressed to circular, convex tablets of suitable crushing strength and disintegration time. Tablet weight is 140 mg and diameter 7 mm.

The core tablets are coated to obtain gastric resistance. The polymer is an aqueous methacrylic acid-ethyl acrylate copolymer dispersion (Eudragit L30 D-55). Talc is added as a glidant to the polymer dispersion, and triethyl citrate functions as plasticizer.

The function of each of the excipients is provided in the table, below.

**Table 3.3: Function of excipients**

| **Ingredient** | **Function** |
|---|---|
| Mannitol | Filler |
| Microcrystalline cellulose | Filler, carrier for API, disintegrant |
| Hypromellose | Binder |
| Magnesium stearate | Lubricant |
| Water, purified* | Solvent |
| Methacrylic acid-ethyl acrylate copolymer (1:1) dispersion 30 per cent | Enteric coating polymer |
| Talc | Glidant |
| Triethyl citrate | Plasticizer |

| | |
|---|---|
| ** Evaporates during the manufacturing process* | |

### 3.4 Analysis of enteric coated composition

A suitable approach for assessing the content and impurities etc. of the enteric coated tablets is as follows. Compound 1A is extracted from the tablets by stirring in sample solvent. After centrifugation, to remove insoluble particles, the amount of 1 A, Individual Related Substances and Total Related Substances in the supernatant may be determined using the instrument conditions described in Table 3.4. The amount of 1A and its related substances are determined by means of reversed phase chromatography and UV detection.

**Table 3.4: HPLC method parameters and solutions for 1A tablets**

| **Method parameters and solutions** | | | |
|---|---|---|---|
| Instument | Waters Alliance Separations Module with Waters 2487 UV detector or equivalent HPLC equipment with binary pump and UV detector | | |
| Column | Waters Atlantis dC18, 150 x 2.1 mm, 3 µ | | |
| Mobile phase A | 0.05 % Trifluoroacetic Acid in Water | | |
| Mobile phase B | 0.05 % Trifluoroacetic Acid in Acetonitrile | | |
| Sample solvent | Mobile Phase A/Mobile Phase B (50/50) | | |
| Injection volume | 50 µL (50 µg 1A tablets), 20 µL (300 µg 1A tablets) | | |
| Column temperature | 25 °C | | |
| Flow rate | 0.3 mL/min | | |
| Detection wavelength | 250 nm | | |
| Run time | 60 min | | |
| Gradient program | **Time (min)** | **% A** | **% B** |
| | 0 | 80 | 20 |
| | 1 | 80 | 20 |
| | 51 | 10 | 90 |
| | 52 | 80 | 20 |
| | 60 | 80 | 20 |
| Typical retention times (under stated HPLC conditions | **Compound** | **Retention time (min)** | **Relative retention time (RRT)** |
| | 1A | 26.4 | 1.00 |
| | reaction product 1B | 34.0 | 1.29 |

A dissolution test may be performed as follows. The dissolution test is divided into two steps: the first step tests the resistance of the enteric coating and the second step tests the dissolution rate. The coating resistance is tested in 0.1 M hydrochloric acid for 3 h. The dissolution is tested in 50 mM Sodium phosphate buffer pH 6.8 during 1 h. Withdrawn samples are centrifuged to remove insoluble particles prior to analysis.

The amount of 1A released from the composition may be determined by means of reversed phase chromatography and UV detection according to Table 3.5.

**Table 3.5: HPLC method parameters and solutions for dissolution test of 1A tablets**

| **Method parameters and solutions** | | | | |
|---|---|---|---|---|
| Dissolution bath | Prolabo Dissolutest | | | |
| Dissolution apparatus | Modified Apparatus I (basket) according to USP. 100 ml vessels | | | |
| Dissolution bath settings | Stirring rate | | 100 ± 2 rpm | |
| | Bath temperature | | 37.0 ± 0.5 °C | |
| | Distance basket to vessel bottom | | 10 ± 1 mm | |
| | | | | |
| Instument | Waters Alliance Separations Module with Waters 2487 UV detector or equivalent HPLC equipment with binary pump and UV detector | | | |
| Column | Waters Atlantis dC18, 150 x 2.1 mm, 3 µ | | | |
| Mobile phase A | 0.05 % Trifluoroacetic Acid in Water | | | |
| Mobile phase B | 0.05 % Trifluoroacetic Acid in Acetonitrile | | | |
| Dissolution medium | Acid stage | | 0.1 M HCl | |
| | Buffer stage | | 50 mM Sodium phosphate buffer pH 6.8 | |
| Injection volume | Acid stage | | 100 µL | |
| | Buffer stage | | 100 µL (50 µg 1A tablets), 15 µL (300 µg 1A tablets) | |
| Column temperature | 25 °C | | | |
| Flow rate Detection wavelength | 0.3 mL/min 250 nm | | | |
| Run time | 60 min | | | |
| Gradient program | **Time (min)** | **% A** | | **% B** |
| | 0 | 65 | | 35 |
| | 19 | 30 | | 70 |
| | 20 | 65 | | 35 |
| | 26 | 65 | | 35 |
| | | | | |
| Typical retention times | **Compound** | **Retention time (min)** | | |
| | 1A | 13.2 | | |

It was found that the release of compound 1A was undetectable following the dissolution test in 0.1M HCl. When the dissolution test was repeated in pH 6.8 media, however, dissolution of the tablets and release of the compound 1A was virtually complete for each tablet tested.

Accordingly, when used *in vivo*, such enteric coated compositions will prevent the active ingredients being exposed, following oral administration, to an acidic nitrite-containing medium in the stomach and thus prevent or significantly reduce the production of potentially genotoxic reaction products of such active ingredients.

### Example 4: Nitration of additional compounds of Formula I.

Tests were carried out on the following additional compounds of Formula I to determine their predisposition to nitration:

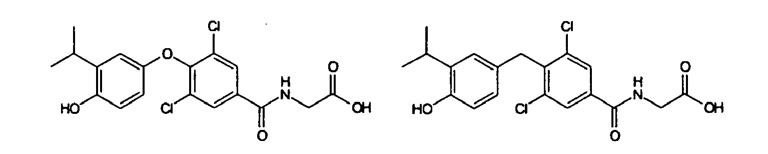

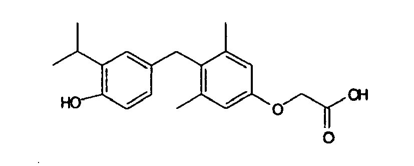

It was found that in all cases exposure to solutions of various concentrations of nitrate and nitrite as in Example 2.4 above resulted in nitration.

### Example 5: Clinical testing

Clinically relevant dosages of compound IA were given to human subjects in the form of a solution in a single dose. Nitrated reaction product was detected in a significant number of the subjects. When the compound was administered in the form of enteric coated tablets, a dramatic reduction in nitrated product was observed.

## Claims

1. A pharmaceutical composition suitable for oral administration, comprising:
(i) a compound of Formula I: 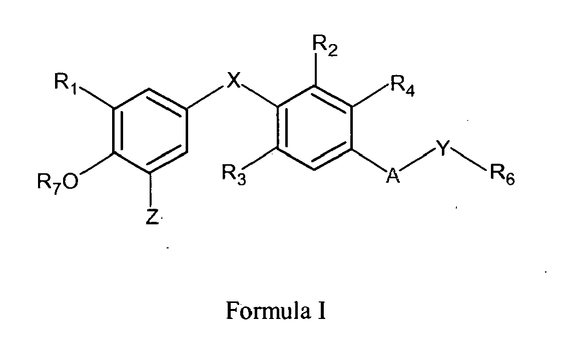 wherein:
Z is H or an alternative group capable of being substituted by NO₂ via a nitrite-based nitration reaction;
R₁ is selected from hydrogen, halogen, trifluoromethyl, or alkyl of 1 to 6 carbons or cycloalkyl of 3 to 7 carbons;
X is oxygen (-O-), sulphur (-S-), carbonyl (-CO-), methylene (-CH₂-), or -NH-;
R₂ and R₃ are the same or different and are hydrogen, halogen, alkyl of 1 to 4 carbons or cycloalkyl of 3 to 6 carbons, at least one of R₂ and R₃ being other than hydrogen;
R₄ is hydrogen or lower alkyl;
A is oxygen (-O-), methylene (-CH₂-), -CONR₅-, -NR₅-, or -NR₅CO-;
R₅ is H or lower alkyl;
R₆ is carboxylic acid (-CO₂H), or an ester thereof;
Y is -(CH₂)ₙ, where n is 0, 1, 2, 3, 4 or 5 and wherein one or more of the CH₂ groups may optionally be substituted with halogen, or Y is -C=C-, which may be *cis* or *trans*; and
R₇ is hydrogen, or an alkanoyl or aroyl group, or other group capable of bioconversion to generate the free phenol structure (wherein R₇ = H);
or a pharmaceutically acceptable salt or ester thereof;
(ii) at least one pharmaceutically-acceptable excipient; and
(iii) an enteric coating.

2. A composition according to claim 1 wherein:
X is oxygen or -CH₂-;
A is -NH-, -O-, -CH₂- or -CONR₅-;
R₁ is H, iodo or isopropyl;
R₂ and R₃ are each independently halogen or alkyl;
R₄ is H or methyl;
Y is -(CH₂)ₙ- and n is 1 or 2;
A is -NR₅CO- and R₅ is H; and
Z is H.

3. A composition according to claim 1 in which compound (i) is selected from 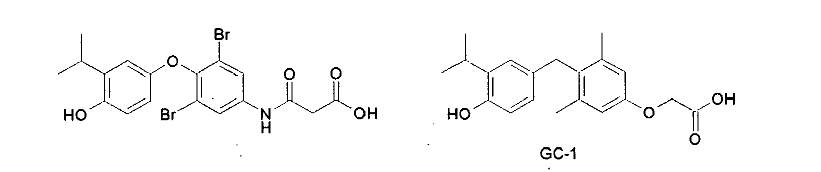 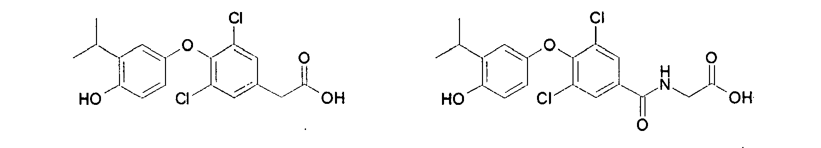 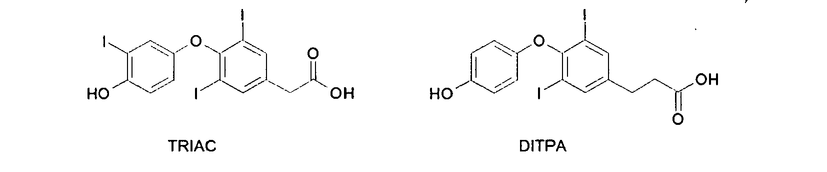 or a pharmaceutically acceptable salt or ester thereof.

4. A composition according to claim 3 in which compound (i) is 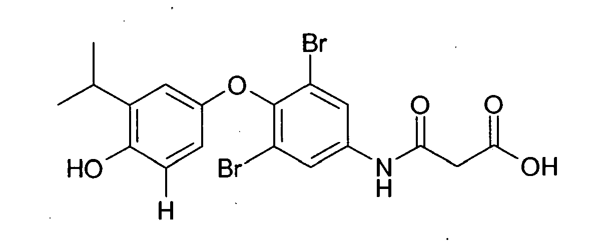 or a pharmaceutically acceptable salt or ester thereof; or 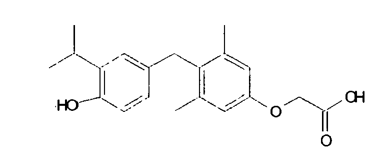 or a pharmaceutically acceptable salt or ester thereof.

5. A composition according to any preceding claim wherein an inert coating is provided between that portion of the composition containing the compound (i), and the enteric coating (iii).

6. A composition according to any preceding claim wherein the enteric coating comprises an acrylate polymer, a methacryate polymer or an acrylate-methacrylate copolymer.

7. A composition according to any preceding claim being in the form of an enteric coated tablet and containing the following ingredients: Mannitol, Microcrystalline cellulose, Hypromellose, magnesium stearate, Water, Methacrylic acid-ethyl acrylate copolymer (1:1), Talc and Triethyl citrate.

8. A composition according to any preceding claim, wherein 5% or less of the compound of Formula I is released in at least one hour, when release is measured in a USP apparatus II in 500ml of simulated gastric fluid or 0.1N HCl at 37°C with a stirring rate of 50 revolutions per minute.

9. A composition according to any preceding claim, also containing an antioxidant, preferably an antioxidant selected from the group consisting of ascorbic acid, fumaric acid, malic acid, propionic acid, or a salt of any of the said acids, monothioglycerol, potassium metabisulphite, sodium bisulphite, sodium sulphite, sodium metabisulphite, α-tocopherol, ascorbyl palmitate, butylated hydroxyanisole, butylated hydroxytoluene, ethyl oleate and propyl gallate.

10. A composition according to claim 9 wherein the compound (i) and the antioxidant are substantially homogeneously mixed.

11. A pharmaceutical composition suitable for oral administration, comprising:
(i) a compound of Formula I: 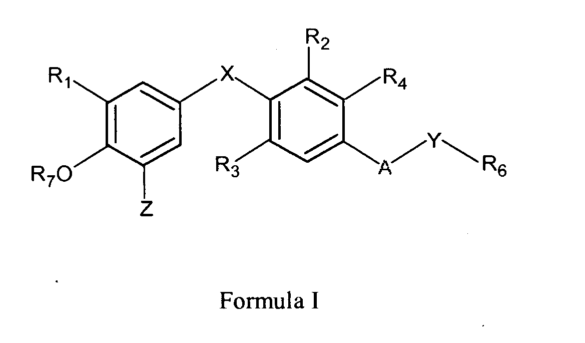 wherein:
Z is H or an alternative group capable of being substituted by NO₂ via a nitrite-based nitration reaction;
R₁ is selected from hydrogen, halogen, trifluoromethyl, or alkyl of 1 to 6 carbons or cycloalkyl of 3 to 7 carbons;
X is oxygen (-O-), sulphur (-S-), carbonyl (-CO-), methylene (-CH₂-), or -NH-;
R₂ and R₃ are the same or different and are hydrogen, halogen, alkyl of 1 to 4 carbons or cycloalkyl of 3 to 6 carbons, at least one of R₂ and R₃ being other than hydrogen;
R₄ is hydrogen or lower alkyl;
A is oxygen (-O-), methylene (-CH₂-), -CONR₅-, -NR₅-, or -NR₅CO-;
R₅ is H or lower alkyl;
R₆ is carboxylic acid, or an ester thereof;
Y is -(CH₂)ₙ, where n is 0, 1, 2, 3, 4 or 5 and wherein one or more of the CH₂ groups may optionally be substituted with halogen, or Y is -C=C-, which may be *cis* or *trans*; and
R₇ is hydrogen, or an alkanoyl or aroyl group, or other group capable of bioconversion to generate the free phenol structure (wherein R₇ = H);
or a pharmaceutically acceptable salt or ester thereof;
(ii) at least one antioxidant; and
(iii) at least one pharmaceutically-acceptable excipient.

12. A composition according to any of claims 9 to 11, wherein the antioxidant is present in an amount of from 0.0001 to 15 mmol per dose.

13. A composition according to any preceding claim also comprising a pharmacologically active ingredient selected from hypolipidaemic agents, antidiabetic agents, antidepressants, bone resorption inhibitors, appetite suppressants and/or anti-obesity agents.

14. A method of stabilising a pharmaceutical composition suitable for oral administration, the pharmaceutical composition comprising:
(i) a compound of Formula I as defined in any one of claims 1 to 4 or a pharmaceutically acceptable salt or ester thereof; and
(ii) at least one pharmaceutically-acceptable excipient;
the method comprising providing the composition with an enteric coating.

15. The use of an enteric coating or an antioxidant or both an enteric coating and an antioxidant for reduction or prevention of nitration of said compound in a pharmaceutical composition suitable for oral administration containing a compound of Formula I as defined in any one of claims 1 to 4 or a pharmaceutically acceptable salt or ester thereof.

16. A composition according to any of claims 1 to 13, for use in therapy.

17. Use of a composition according to any of claims 1 to 13 in the preparation of a medicament for preventing, inhibiting or treating a disease selected from obesity, hypercholesterolemia, dyslipidaemia, atherosclerosis, cardiac arrhythmias, depression, osteoporosis, hypothyroidism, goitre, thyroid cancer, glaucoma and congestive heart failure.

## Patentansprüche

1. Pharmazeutische Zusammensetzung, die sich zur oralen Verabreichung eignet, umfassend:
(i) eine Verbindung der Formel I: 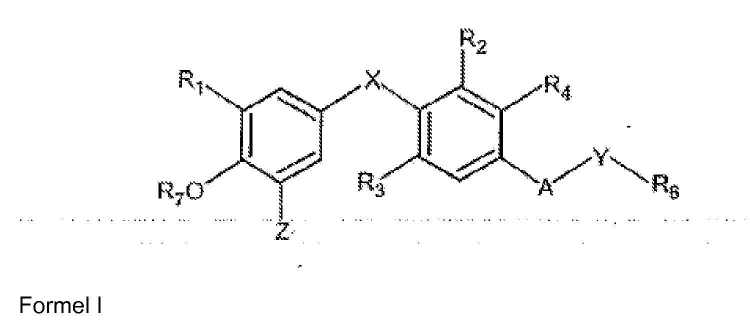 worin:
Z H oder eine alternative Gruppe, die über eine nitrit-basierte Nitrierungsreaktion mit NO₂ substituiert werden kann, ist
R₁ aus Wasserstoff, Halogen, Trifluormethyl oder Alkyl mit 1 bis 6 Kohlenstoffen oder Cycloalkyl mit 3 bis 7 Kohlenstoffen ausgewählt ist;
X Sauerstoff (-O-), Schwefel (-S-), Carbonyl (-CO-), Methylen (-CH₂-) oder-NH- ist;
R₂ und R₃ gleich oder verschieden sind und Wasserstoff, Halogen, Alkyl mit 1 bis 4 Kohlenstoffen oder Cycloalkyl mit 3 bis 6 Kohlenstoffen sind, wobei mindestens eine der Gruppen R₂ und R₃ von Wasserstoff verschieden ist;
R₄ Wasserstoff oder ein niederes Alkyl ist;
A Sauerstoff (-O-), Methylen (-CH₂-), -CONR₅-, -NR₅- oder -NR₅CO- ist;
R₅ H oder ein niederes Alkyl ist;
R₆ Carbonsäure (-CO₂H) oder ein Ester davon ist;
Y -(CH₂)ₙ ist, wobei n 0, 1, 2, 3, 4 oder 5 ist und wobei eine oder mehrere der CH₂-Gruppen wahlweise mit einem Halogen substituiert sein können, oder Y -C=C- ist, das *cis* oder *trans* sein kann; und
R₇ Wasserstoff oder eine Alkanoyl- oder Aroylgruppe oder eine andere Gruppe, die mittels Biokonversion die freie Phenolstruktur (mit R₇ = H) bilden kann, ist;
oder ein pharmazeutisch akzeptables Salz oder einen pharmazeutisch akzeptablen Ester davon;
(ii) mindestens einen pharmazeutisch akzeptablen Arzneimittelträger; und
(iii) einen magensaftresistenten Überzug.

2. Zusammensetzung nach Anspruch 1, bei der
X Sauerstoff oder -CH₂- ist;
A -NH-, -O-, -CH₂- oder -CONR₅- ist;
R₁ H, Iod oder Isopropyl ist;
R₂ und R₃ jeweils unabhängig voneinander Halogen oder Alkyl sind;
R₄ H oder Methyl ist;
Y -(CH₂)ₙ- ist und n 1 oder 2 ist;
A -NR₅CO- ist und R₅ H ist; und
Z H ist.

3. Zusammensetzung nach Anspruch 1, bei der Verbindung (i) aus 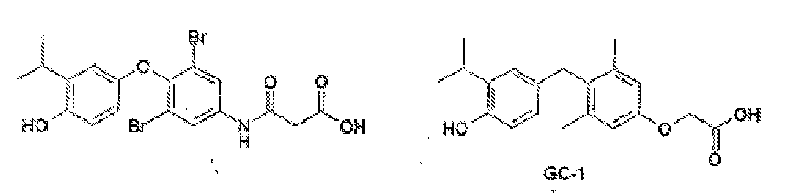 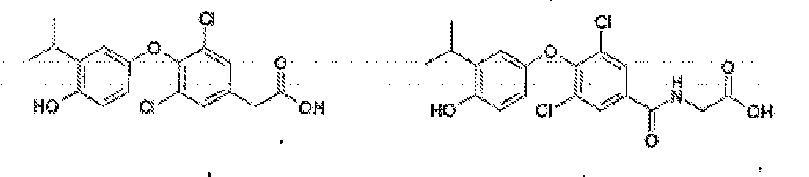 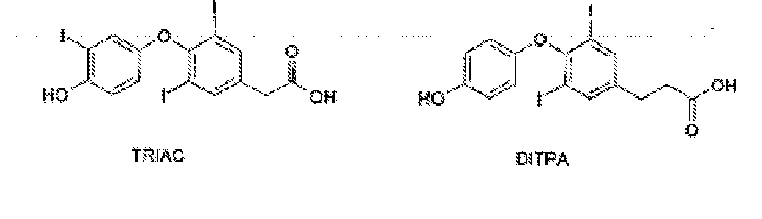 oder einem pharmazeutisch akzeptablen Salz davon oder einem pharmazeutisch akzeptablen Ester davon ausgewählt ist.

4. Zusammensetzung nach Anspruch 3, bei der Verbindung (i) aus 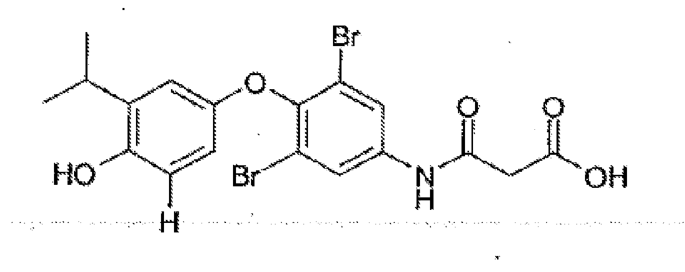 oder einem pharmazeutisch akzeptablen Salz davon oder einem pharmazeutisch akzeptablen Ester davon; oder aus 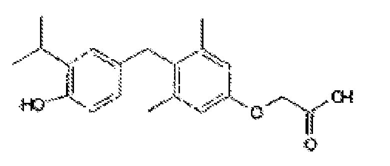 oder einem pharmazeutisch akzeptablen Salz davon oder einem pharmazeutisch akzeptablen Ester davon ausgewählt ist.

5. Zusammensetzung nach einem der vorangegangenen Ansprüche, bei der zwischen dem Anteil der Zusammensetzung, der die Verbindung (i) enthält, und dem magensaftresistenten Überzug (iii) eine inerte Beschichtung bereitgestellt wird.

6. Zusammensetzung nach einem der vorangegangenen Ansprüche, bei der der magensaftresistente Überzug ein Acrylatpolymer, ein Methacrylatpolymer oder ein Acrylat-Methacrylat-Copolymer umfasst.

7. Zusammensetzung nach einem der vorangegangenen Ansprüche, die die Form einer magensaftresistent überzogenen Tablette besitzt und folgende Bestandteile enthält: Mannitol, mikrokristalline Cellulose, Hypromellose, Magnesiumstearat, Wasser, Methacrylsäure-Ethylacrylat-Copolymer (1:1), Talkum und Triethylcitrat.

8. Zusammensetzung nach einem der vorangegangenen Ansprüche, bei der 5% oder weniger der Verbindung von Formel I innerhalb von mindestens einer Stunde freigesetzt werden, wobei die Freisetzung mittels einer USP-Vorrichtung II in 500 ml künstlichem Magensaft oder 0,1 N HCl bei 37 °C und einer Rührrate von 50 Umdrehungen pro Minute gemessen wird.

9. Zusammensetzung nach einem der vorangegangenen Ansprüche, die weiterhin ein Antioxidans, vorzugsweise ein aus der Gruppe bestehend aus Ascorbinsäure, Fumarsäure, Apfelsäure, Propionsäure oder einem Salz dieser Säuren, Monothioglycerol, Kaliummetabisulfit, Natriumbisulfit, Natriumsulfit, Natriummetabisulfit, α-Tocopherol, Ascorbylpalmitat, butyliertem Hydroxyanisol, butyliertem Hydroxytoluol, Ethyloleat und Propylgallat ausgewähltes Antioxidans enthält.

10. Zusammensetzung nach Anspruch 9, bei der die Verbindung (i) und das Antioxidans im Wesentlichen homogen gemischt sind.

11. Pharmazeutische Zusammensetzung, die sich zur oralen Verabreichung eignet, umfassend:
(i) eine Verbindung der Formel I: 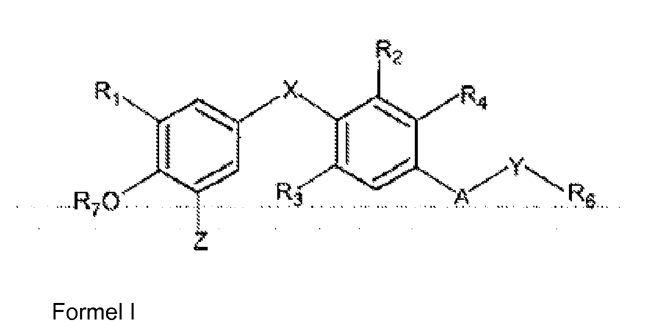 worin:
Z H oder eine alternative Gruppe, die über eine nitrit-basierte Nitrierungsreaktion mit NO₂ substituiert werden kann, ist
R₁ aus Wasserstoff, Halogen, Trifluormethyl oder Alkyl mit 1 bis 6 Kohlenstoffen oder Cycloalkyl mit 3 bis 7 Kohlenstoffen ausgewählt ist;
X Sauerstoff (-O-), Schwefel (-S-), Carbonyl (-CO-), Methylen (-CH₂-) oder -NH- ist;
R₂ und R₃ gleich oder verschieden sind und Wasserstoff, Halogen, Alkyl mit 1 bis 4 Kohlenstoffen oder Cycloalkyl mit 3 bis 6 Kohlenstoffen sind, wobei mindestens eine der Gruppen R₂ und R₃ von Wasserstoff verschieden ist;
R₄ Wasserstoff oder ein niederes Alkyl ist;
A Sauerstoff (-O-), Methylen (-CH₂-), -CONR₅-, -NR₅- oder -NR₅CO- ist;
R₅ H oder ein niederes Alkyl ist;
R₆ Carbonsäure oder ein Ester davon ist;
Y -(CH₂)ₙ ist, wobei n 0, 1, 2, 3, 4 oder 5 ist und wobei eine oder mehrere der CH₂-Gruppen wahlweise mit einem Halogen substituiert sein können, oder Y -C=C- ist, das *cis* oder *trans* sein kann; und
R₇ Wasserstoff oder eine Alkanoyl- oder Aroylgruppe oder eine andere Gruppe, die mittels Biokonversion die freie Phenolstruktur (mit R₇ = H) bilden kann, ist;
oder ein pharmazeutisch akzeptables Salz oder einen pharmazeutisch akzeptablen Ester davon;
(i) mindestens ein Antioxidans; und
(ii) mindestens einen pharmazeutisch akzeptablen Arzneimittelträger.

12. Zusammensetzung nach einem der Ansprüche 9 bis 11, bei der das Antioxidans in einer Menge von 0,0001 bis 15 mmol pro Dosis vorliegt.

13. Zusammensetzung nach einem der vorangegangenen Ansprüche, die weiterhin einen pharmazeutisch wirksamen Bestandteil umfasst, der aus hypolipidämischen Agentien, antidiabetischen Agentien, Antidepressiva, Knochenresporptionsinhibitoren, Appetitzüglern und/oder Antiadiposita-Agentien ausgewählt ist.

14. Verfahren zur Stabilisierung einer pharmazeutischen Zusammensetzung, die sich zur oralen Verabreichung eignet, wobei die pharmazeutische Zusammensetzung Folgendes umfasst:
(i) eine Verbindung der Formel I, wie in einem der Ansprüche 1 bis 4 definiert, oder ein pharmazeutisch akzeptables Salz oder einen pharmazeutisch akzeptablen Ester davon; und
(ii) mindestens einen pharmazeutisch akzeptablen Arzneimittelträger;
wobei das Verfahren die Beschichtung der Zusammensetzung mit einem magensaftresistenten Überzug umfasst.

15. Verwendung eines magensaftresistenten Überzugs, oder eines Antioxidans oder eines magensaftresistenten Überzugs und eines Antioxidans zur Reduktion oder Prävention einer Nitrierung der Verbindung in einer pharmazeutischen Zusammensetzung, die sich für eine orale Verabreichung eignet und eine Verbindung der Formel I, wie sie in einem der Ansprüche 1 bis 4 definiert ist, oder ein pharmazeutisch akzeptables Salz oder einen pharmazeutisch akzeptabler Ester davon enthält.

16. Zusammensetzung nach einem der Ansprüche 1 bis 13 zur Anwendung bei der Therapie.

17. Verwendung einer Zusammensetzung nach einem der Ansprüche 1 bis 13 bei der Herstellung eines Medikaments zur Prävention, Inhibition oder Behandlung einer Erkrankung, die aus Adipositas, Hypercholesterinämie, Dyslipidämie, Atherosklerose, Herzrhythmusstörungen, Depression, Osteoporose, Hypothyreose, Struma, Schilddrüsenkrebs, Glaukom oder kongestive Herzinsuffizienz ausgewählt ist.

## Revendications

1. Composition pharmaceutique appropriée pour une administration par voie orale, comprenant :
(i) un composé de formule I : 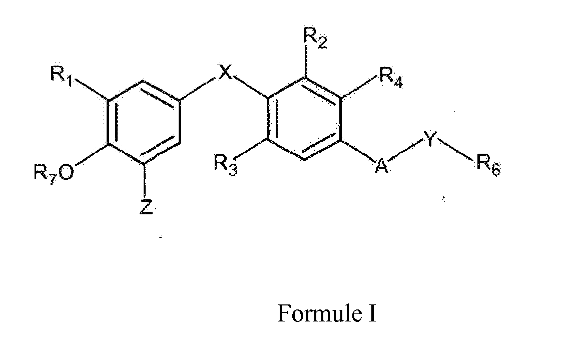 où :
Z est un H ou un groupe en variante capable d'être substitué par NO₂ au moyen d'une réaction de nitration à base de nitrite ;
R₁ est choisi parmi un hydrogène, un halogène, un trifluorométhyle, ou un alkyle de 1 à 6 carbones ou cycloalkyle de 3 à 7 carbones ;
X est un oxygène (-O-), un soufre (-S-), un carbonyle (-CO-), un méthylène (-CH₂-), ou -NH-;
R₂ et R₃ sont identiques ou différents et sont un hydrogène, un halogène, un alkyle de 1 à 4 carbones ou cycloalkyle de 3 à 6 carbones, au moins l'un parmi R₂ et R₃ étant autre chose qu'un hydrogène ;
R₄ est un hydrogène ou un alkyle inférieur ;
A est un oxygène (-O-), un méthylène (-CH₂-), -CONR₅-, -NR₅-, ou -NR₅CO-;
R₅ est un H ou un alkyle inférieur ;
R₆ est un acide carboxylique (-CO₂H), ou un ester de celui-ci ;
Y est -(CH₂)ₙ, où n est 0, 1, 2, 3, 4 ou 5 et où un ou plusieurs des groupes CH₂ peut éventuellement être substitué avec un halogène, ou Y est -C=C-, qui peut être *cis* ou *trans* ; et
R₇ est un hydrogène, ou un groupe alcanoyle ou aroyle, ou un autre groupe capable de bioconversion pour générer la structure phénolique libre (où R₇ = H) ;
ou un sel ou ester pharmaceutiquement acceptable de celui-ci ;
(ii) au moins un excipient pharmaceutiquement acceptable ; et
(iii) un revêtement gastro-résistant.

2. Composition selon la revendication 1, où :
X est un oxygène ou -CH₂-;
A est -NH-, -O-, -CH₂- ou -CONR₅-;
R₁ est un H, iodo ou isopropyle ;
R₂ et R₃ sont chacun indépendamment un halogène ou un alkyle,
R₄ est un H ou un méthyle ;
Y est -(CH₂)ₙ- et n est 1 ou 2 ;
A est -NR₅CO- et R₅ est un H ; et
Z est un H.

3. Composition selon la revendication 1 dans laquelle le composé (i) est choisi parmi 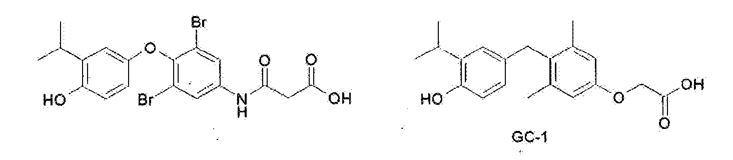 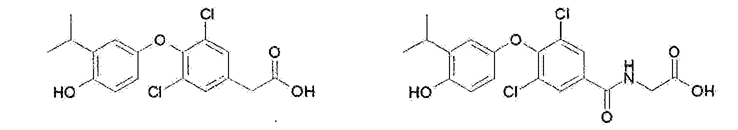 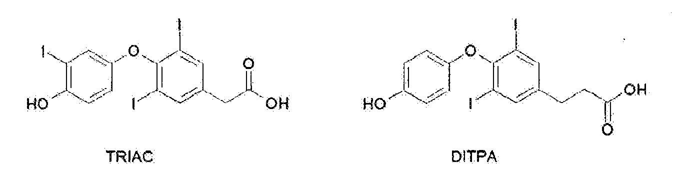 ou un sel ou ester pharmaceutiquement acceptable de ces derniers.

4. Composition selon la revendication 3 dans laquelle le composé (i) est 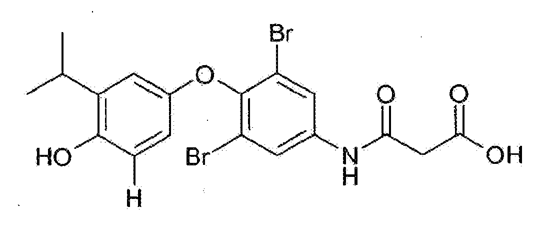 ou un sel ou ester pharmaceutiquement acceptable de celui-ci ; ou 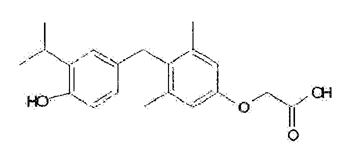 ou un sel ou ester pharmaceutiquement acceptable de celui-ci.

5. Composition selon l'une quelconque des revendications précédentes, où un revêtement inerte est prévu entre cette partie de la composition contenant le composé (i), et le revêtement gastro-résistant (iii).

6. Composition selon l'une quelconque des revendications précédentes, où le revêtement gastro-résistant comprend un polymère d'acrylate, un polymère de méthacrylate ou un copolymère d'acrylate-méthacrylate.

7. Composition selon l'une quelconque des revendications précédentes sous la forme d'un comprimé à revêtement gastro-résistant et contenant les ingrédients suivants : mannitol, cellulose microcristalline, hypromellose, stéarate de magnésium, eau, copolymère d'acide méthacrylique- acrylate d'éthyle (1: 1), talc et citrate de triéthyle.

8. Composition selon l'une quelconque des revendications précédentes, où 5 % au moins du composé de formule I est libéré en au moins une heure, la libération étant mesurée dans un appareil USP II dans 500 ml de fluide gastrique simulé ou HCl 0,1 N à 37°C avec une vitesse d'agitation de 50 tr/min.

9. Composition selon l'une quelconque des revendications précédentes, contenant également un antioxydant, de préférence un antioxydant choisi parmi le groupe consistant en acide ascorbique, acide fumarique, acide malique, acide propionique, ou un sel de l'un quelconque desdits acides, monothioglycérol, métabisulfite de potassium, bisulfite de sodium, sulfite de sodium, métabisulfite de sodium, α-tocophérol, palmitate d'ascorbyle, hydroxyanisole butylé, hydroxytoluène butylé, oléate d'éthyle et propyl gallate.

10. Composition selon la revendication 9, où le composé (i) et l'antioxydant sont mélangés de manière sensiblement homogène.

11. Composition pharmaceutique appropriée pour une administration par voie orale, comprenant :
(i) un composé de formule I : 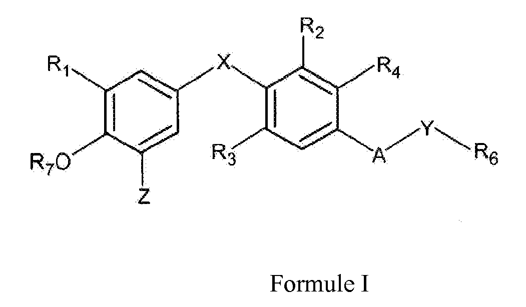 où :
Z est un H ou un groupe en variante capable d'être substitué par NO₂ au moyen d'une réaction de nitration à base de nitrite ;
R₁ est choisi parmi un hydrogène, un halogène, un trifluorométhyle, ou un alkyle de 1 à 6 carbones ou cycloalkyle de 3 à 7 carbones ;
X est un oxygène (-O-), un soufre (-S-), un carbonyle (-CO-), un méthylène (-CH₂-), ou -NH-;
R₂ et R₃ sont identiques ou différents et sont un hydrogène, un halogène, un alkyle de 1 à 4 carbones ou cycloalkyle de 3 à 6 carbones, au moins l'un parmi R₂ et R₃ étant autre chose qu'un hydrogène ;
R₄ est un hydrogène ou un alkyle inférieur ;
A est un oxygène (-O-), un méthylène (-CH₂-), -CONR₅-, -NR₅-, ou -NR₅CO- ;
R₅ est un H ou un alkyle inférieur ;
R₆ est un acide carboxylique, ou un ester de celui-ci ;
Y est -(CH₂)ₙ, où n est 0, 1, 2, 3, 4 ou 5 et où un ou plusieurs des groupes CH₂ peut éventuellement être substitué avec un halogène, ou Y est -C=C-, qui peut être *cis* ou *trans* ; et
R₇ est un hydrogène, ou un groupe alcanoyle ou aroyle, ou un autre groupe capable de bioconversion pour générer la structure phénolique libre (où R₇ = H);
ou un sel ou ester pharmaceutiquement acceptable de celui-ci ;
(ii) au moins un antioxydant ; et
(iii) au moins un excipient pharmaceutiquement acceptable.

12. Composition selon l'une quelconque des revendications 9 à 11, où l'antioxydant est présent dans une quantité de 0,0001 à 15 mmoles par dose.

13. Composition selon l'une quelconque des revendications précédentes comprenant également un ingrédient pharmacologiquement actif choisi parmi des agents hypolipidémiants, des agents antidiabétiques, des antidépresseurs, des inhibiteurs de résorption osseuse, des suppresseurs d'appétit et/ou des agents anti-obésité.

14. Procédé de stabilisation d'une composition pharmaceutique appropriée pour une administration par voie orale, la composition pharmaceutique comprenant :
(i) un composé de formule 1 tel que défini selon l'une quelconque des revendications 1 à 4 ou un sel ou ester pharmaceutiquement acceptable de celui-ci ; et
(ii) au moins un excipient pharmaceutiquement acceptable ;
le procédé consistant à fournir à la composition un revêtement gastro-résistant.

15. Utilisation d'un revêtement gastro-résistant ou d'un antioxydant ou à la fois d'un revêtement gastro-résistant et d'un antioxydant pour la réduction ou la prévention de la nitration dudit composé dans une composition pharmaceutique appropriée pour une administration par voie orale contenant un composé de formule 1 tel que défini selon l'une quelconque des revendications 1 à 4 ou un sel ou ester pharmaceutiquement acceptable de celui-ci.

16. Composition selon l'une quelconque des revendications 1 à 13, destinée à une utilisation en thérapie.

17. Utilisation d'une composition selon l'une quelconque des revendications 1 à 13 dans la préparation d'un médicament pour prévenir, inhiber ou traiter une maladie choisie parmi l'obésité, l'hypercholestérolémie, la dyslipidémie, l'athérosclérose, l'arythmie cardiaque, la dépression, l'ostéoporose, l'hypothyroïdie, le goitre, le cancer de la thyroïde, le glaucome et l'insuffisance cardiaque congestive.
